# EUROPÄISCHE PATENTSCHRIFT

(11) **EP 1 802 376 B1**
(45) Veröffentlichungstag und Bekanntmachung des Hinweises auf die Patenterteilung: **27.05.2009**
(21) Anmeldenummer: 05716980.7
(22) Anmeldetag: 10.03.2005
(51) Int. Cl.: A61Q 15/00, A61K 8/26, A61K 8/28, A61K 8/365

(54) **TRANSPARENTE KOSMETISCHE ODER DERMATOLOGISCHE FORMULIERUNG**
TRANSPARENT COSMETIC OR DERMATOLOGICAL FORMULATION
FORMULATION COSMETIQUE ET DERMATOLOGIQUE TRANSPARENTE

(30) Priorität: 27.04.2004 DE 102004020711
(43) Veröffentlichungstag der Anmeldung: 04.07.2007
(73) Patentinhaber: Beiersdorf AG, 20253 Hamburg (DE)
(72) Erfinder: SCHULZ, Ulrike, 20253 Hamburg (DE); CHRIST, Gordon, 22763 Hamburg (DE)
(86) Internationale Anmeldenummer: PCT/EP2005/051068
(87) Internationale Veröffentlichungsnummer: WO 2005/105026

(56) Entgegenhaltungen:
- DE-A1- 19 519 404
- DE-A1- 19 857 235
- GB-A- 1 541 396
- GB-A- 2 280 111
- US-A- 3 509 253
- US-A1- 2003 065 027

## Beschreibung

Die Erfindung betrifft eine klare, kosmetische und dermatologische Formulierung mit reduzierter Klebrigkeit.

Vor allem aus ästhetischen Gründen werden transparente und transluzente Produkte von vielen Verbrauchern bevorzugt. Transparente Formulierungen kommen so z. B. häufig als Deo oder Antitranspirant (AT) zum Einsatz. Diese lassen sich heutzutage durch folgende Technologien realisieren:
1. wässrig-alkoholische Formulierungen
2. Wasser-in-Silikon-Emulsionen
3. Mikro-Emulsionen

Die wässrig alkoholischen Deo- und AT-Formulierungen basieren zumeist auf Wasser und Alkohol als Medium, Deo- und Antitranspirantmittel als Wirkstoffe sowie Parfüm. Löslichkeitsvermittler und Verdicker (zumeist auf Kohlenhydratbasis) als zusätzliche Agenzien. Sie werden vom Verbraucher als frisch und kühlend empfunden, sind aber gleichzeitig mit einer ganzen Reihe an Nachteilen behaftet. So ist beispielsweise die Applikation vor allem auf frisch rasierter Haut durch den Alkoholgehalt mit Unverträglichkeiten verbunden. Ein weiterer großer Nachteil ist die Tatsache, dass in derartige Systeme keine größeren Ölmengen eingearbeitet werden können. Durch den für eine hocheffektive Wirkleistung erforderlichen hohen Gehalt an Antitranspirantsalz verbleibt nach der Applikation auf der Haut ein weißer Rückstand, der vom Verbraucher als überaus störend empfunden wird. Durch die technologisch bedingte Abwesenheit einer ausreichend großen Ölphase kann dieser allerdings nicht kaschiert werden. Darüber hinaus führt die Verwendung von Kohlenhydrat-Verdickern zu einer hohen Klebrigkeit des Produktes nach dem Verdunsten des Alkohols.

Wasser-in-Silikon-Emulsionen gehören zur Gruppe der Wasser-in-Öl-Emulsionen. Die Wasserphase, enthaltend Ethanol oder mehrwertige Alkohole wie beispielsweise Propylen Glycol und wasserlösliche Wirkstoffe wie AT-Mittel und/oder Deowirker, nimmt etwa 75-90% der Formulierung ein. Die Ölphase besteht aus einem flüchtigem und einem nicht-flüchtigen Silikonöl sowie einem Silikonemulgator.

Die Transparenz von Wasser-in-Silikon-Emulsionen basiert auf Angleichung der Brechungsindices beider Phasen. Nachteilig ist, dass schon eine z.B. durch Verdunstung bedingte Abweichung der Indices um 0,0004 zu Eintrübungen führt. WO 98/32418 und WO 92/05767 beschreiben derartige Deo- bzw. AT-Formulierungen auf W/Si-Emulsionsbasis.

Ein Ansatz zur Lösung der geschilderten Nachteile ist durch kosmetisch ansprechende alkoholfreie und transparente Produkte möglich geworden, die auf so genannten Mikroemulsionen basieren. Diese haben den großen Vorteil, dass man auch größere Mengen an verschiedenen Ölen mit all den beschriebenen positiven Effekten für den Verbraucher - stabil einarbeiten kann. Formulierungen dieser Art sind prinzipiell mittels Phaseninversionstemperatur-Technologie (PIT) oder Hochdruckhomogenisierung zugänglich. Die notwendige Stabilität des Emulgatorsystems gegenüber hohen Konzentrationen an Antitranspirantsalzen stellt jedoch hohe Anforderungen an die Formulierungskunst des Produktentwicklers.
WO 98/15255 beschreibt Mikroemulsionen. Nachteilig ist jedoch auch bei diesen Formulierungen ein durch den Verdicker bedingtes klebriges Hautgefühl und eine fehlende Fließgrenze.

GB-A-2280111 beschreibt wässrige und klare Antitranspirantzubereitungen in Gelform umfassend Antiranspirantwirkstoffe und Wasser.
US-A-2003065027 offenbart klare Zubereitungen umfassend Parfum, Zitronensäure und Wasser.
US-A-3509253 offenbart klare Antitranspirantzubereitungen umfassend einen Antitranspirantwirkstoff, Weinsäure un Wasser.
DE-A-19857235 beschreibt eine gelförmige Roll-on Formulierung umfassend einen Antitranspirantwirkstoff, Zitronensäure und Wasser.
DE-A-19519404 beschreibt transparente Deodorantzubereitungen umfassend neben dem Deodorantwirkstoff auch Zitronensäure und Wasser.
GB-A-1541396 offenbart transparente Deodorantien umfassend neben Deodorantwirkstoff verschiedenen a-Hydroxysäuren, wie Wein-, Zitronen-, Apfel- oder Milchsäure.
Allen transparenten Gelen des Standes der Technik ist gemein, dass sie zur Gelbildung einen Gelbildner benötigen.

Aufgabe der vorliegenden Erfindung ist es, eine kosmetische Zubereitung bereit zu stellen, die den Stand der Technik bereichert und deren Nachteile vermeiden hilft.

Insbesondere ist es die Aufgabe der vorliegenden Erfindung eine kosmetische und/oder dermatologische Formulierungen bereit zu stellen, die transparent ist und sich durch eine minimierte Klebrigkeit auszeichnet. Insbesondere bestand die Aufgabe darin eine Deo- oder Antitranspirantformulierung bereit zu stellen, die transparent ist und keinerlei Eintrübung aufweist, die sich durch eine minimierte Klebrigkeit auszeichnet und die eine definierte Fließgrenze zur optimierten Ausbringung und Applikation besitzt.

Gelöst wird das Bündel an Aufgaben durch eine kosmetische Formulierung entsprechend Anspruch 1. Gegenstand der Unteransprüche sind vorteilhafte Ausführungsformen der erfindungsgemäßen Zubereitung. Des weiteren umfasst die Erfindung die Verwendung derartiger Zubereitungen.

Es war überraschend und für den Fachmann nicht vorauszusehen, dass eine kosmetische Formulierung umfassend mindestens einen Antitranspirantwirkstoff, Mandelsäure und Wasser zu einer transparenten, viskosen bis pastösen Formulierung geliert und die Bereitstellung einer transparenten und wenig klebrigen kosmetischen Antitranspirant- bzw. Deodorantzubereitung ermöglicht.

Durch die überraschend einfache Kombination von Antitranspirantwirkstoffen und Mandelsäure in Wasser lassen sich transparente kosmetische und dermatologische Formulierungen herstellen, die keinerlei objektiv als auch subjektiv empfundene Klebrigkeit aufweisen.

Die Hydroxyphenylessigsäure oder auch Phenylglykolsäure mit der Formel H₅C₆ -CH(OH) -COOH, C₈H₈O₃, ist bekannt unter dem Namen Mandelsäure. Die Mandelsäure ist gut löslich in Wasser, Alkohol, Ether u. 2-Propanol. Synthetisch erhält man die (±)-Mandelsäure aus Benzaldehyd und Blausäure über das α-Hydroxynitril (Cyanohydrin) und dessen saure Hydrolyse entsprechend Abbildung 2:

### Abbildung 1: Herstellung Mandelsäure

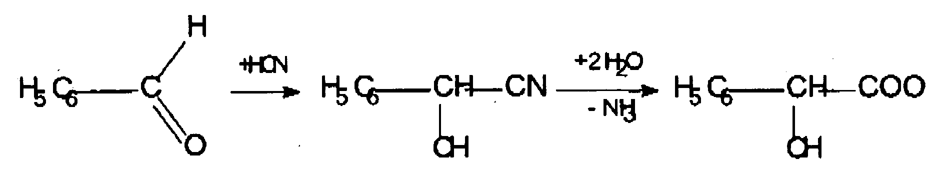

Mittels der Mandelsäure, läßt sich überraschenderweise eine AT- bzw. Deodorantzubereitung herstellen, die die geforderten Eigenschaften, wie Transparenz und geringe Klebrigkeit und darüber hinaus auch die Einstellung einer bestimmten Fließgrenze der Zubereitung ermöglicht. Des weiteren zieht die erfindungsgemäße Formulierung sehr schnell ohne Rückstände zu hinterlassen in die Haut ein.

Die Fließgrenze oder Fließpunkt ist eine Bezeichnung für die kleinste Schubspannung, oberhalb derer ein plastischer Stoff sich rheologisch wie eine Flüssigkeit verhält (DIN 1342-1: 1983-10). Die Bestimmung der Fließgrenze erfolgt durch Aufnahme einer Fließkurve (DIN 53019: 1980-05: DIN 53214: 1982-02). Der erhaltene Wert hängt stark von der Zeitskala (Belastungsrate) ab, die der Messung zugrunde liegt. Dies ist unabhängig davon, ob die Messung mit einem schubspannungs- oder drehzahlgesteuerten Viskosimeter erfolgt. Kurze Zeitskalen (schnelle Belastungen) ergeben in der Regel höhere Werte für die Fließgrenze. Eine zu hohe Fließgrenze kann Ursache von Verlaufstörungen sein. Andererseits lässt sich mit geeignet bemessener Fließgrenze die Neigung der flüssigen Formulierung zum Ablaufen unterdrücken.

Die erfindungsgemäße Zubereitung liegt daher vorteilhaft als Gel- bzw. Hydrogel vor und weist eine Fließgrenze auf, wodurch die Ausbringung und Applikation gegenüber den Zubereitungen aus dem Stand der Technik verbessert ist.
Die erfindungsgemäße Kombination aus AT-Wirkstoff, Mandelsäure, und Wasser ermöglicht über einen einzigartigen Verdickungsmechanismus die Herstelltung einer transparenten kosmetischen Zubereitung. Der Anwender hat somit erstmalig eine wasserklare und dennoch überaus wirksame Zubereitung zur Hand. Die erfindungsgemäße Zubereitung ist in Gelform bequem zu applizieren und weist eine angenehmes Hautgefühl aufgrund der fehlenden Klebrigkeit auf.

Als Antitranspirantwirkstoff lassen sich vorteilhaft saure Aluminium- und/oder Aluminium/Zirkoniumsalze in wässriger Lösung einarbeiten. Hierbei beziehen sich die beschriebenen Konzentrationsbereiche auf die so genannten Aktivgehalte der Antitranspirant-Komplexe: bei den Aluminium-Verbindungen auf wasserfreie Komplexe, bei den Aluminium/Zirkonium-Verbindungen auf wasser- und pufferfreie Komplexe. Als Puffer wird hier üblicherweise Glycin verwendet.

Die nachfolgende Auflistung vorteilhaft einzusetzender Antitranspirant-Wirker soll in keiner Weise einschränkend sein:

Aluminium-Salze (der empirischen Summenformel [Al₂(OH)ₘClₙ], wobei m+n=6):
□ Aluminiumchlorhydrat [Al₂(OH)₅Cl] x H₂O
   Standard Al-Komplexe: Locron L, Locron LIC, Locron LIF (Clariant), Chlorhydrol (Reheis), ACH-303 (Summit), Aloxicoll L (Giulini).
   Aktivierte Al-Komplexe: Reach 501 (Reheis), Aloxicoll 51 L
□ Aluminiumsesquichlorhydrat [Al₂(OH)_{4,5}Cl_{1,5}] x H₂O
   Standard Al-Komplexe: Aloxicoll 31 L (Giulini), Westchlor 186 (Westwood Chemicals)
   Aktivierte Al-Komplexe: Reach 301 (Reheis)
□ Aluminiumdichlorhydrat [Al₂(OH)₄Cl₂] x H₂O

Aluminium-Zirkonium-Salze:
□ Aluminium/Zirkonium Trichlorhydrex Glycin [Al₄Zr(OH)₁₃Cl₃] x H₂O x Gly
   Standard AI/Zr-Komplexe: Rezal 33GC (Reheis), AZG-7164 (Summit)
□ Aluminium/Zirkonium Tetrachlorhydrex Glycin [Al₄Zr(OH)₁₂Cl₄] x H₂O x Gly
   Standard Al/Zr-Komplexe: Rezal 36, Rezal 36G, Rezal 36 GC (Reheis), AZG-368 (Summit), Zirkonal L435G (Giulini), Westchlor ZR 35 BX5, Westchlor ZR 41 (Westwood Chemicals)
□ Aluminium/Zirkonium Pentachlorhydrex Glycin [Al₈Zr(OH)₂₃Cl₅] x H₂O x Gly
   Standard Al/Zr-Komplexe: Rezal 67 (Reheis), Zirkonal L540, Zirkonal L530 PG (Giulini). Westchlor ZR 80B (Westwood Chemicals)
□ Aluminium/Zirkonium Octachlorhydrex Glycin [Al₈Zr(OH)₂₀Cl₈] x H₂O x Gly:
   Westchlor ZR 82B

Ebenso vorteilhaft können aber auch Glycin-freie Aluminium/Zirkonium-Salze eingesetzt werden.

Die Antitranspirant-Wirkstoffe werden in den erfindungsgemäßen Formulierungen in einer Menge von 1 bis 35 Gew.%, vorzugsweise von 1 bis 20 Gew. %, eingesetzt.

Vorteilhaft können erfindungsgemäßen Zubereitungen auch Desodorantien zugesetzt werden. Den üblichen kosmetischen Desodorantien liegen unterschiedliche Wirkprinzipien zugrunde.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. Der Schweißfluss selbst wird dadurch nicht beeinflusst, im Idealfalle wird nur die mikrobielle Zersetzung des Schweißes zeitweilig gestoppt. Auch die Kombination von Adstringentien mit antimikrobiell wirksamen Stoffen in ein und derselben Zusammensetzung ist gebräuchlich.

Alle für Desodorantien gängigen Wirkstoffe können vorteilhaft genutzt werden, beispielsweise Geruchsüberdecker wie die gängigen Parfümbestandteile, Geruchsabsorber, beispielsweise die in der DE 40 09 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäßen Zubereitungen eingearbeitet zu werden. Vorteilhafte Substanzen sind zum Beispiel 2,4,4'-Trichlor-2'-hdroxydiphenylether (Irgasan), 1,6-Di-(4-chlorphenylbiguanido)-hexan (Chlorhexidin), 3,4,4'-Tichlorcarbanilid, quaternäre Ammoniumverbindungen, Nelkenöl, Minzöl, Thymianöl, Triethylcitrat, Farnesol (3,7,11-Trimethyl-2,6,10-dodecatriën-1-ol) sowie die in den DE 37 40 186, DE 39 38 140, DE 42 04 321, DE 42 29 707, DE 42 29 737, DE 42 37 081, DE 43 09 372, DE 43 24 219 beschriebenen wirksamen Agenzien. Auch Natriumhydrogencarbonat ist vorteilhaft zu verwenden.

Die Menge der Desodorantien (eine oder mehrere Verbindungen) in den Zubereitungen beträgt vorzugsweise 0,01 bis 10 Ges.%, bevorzugt 0,05 bis 5 Gew.% bezogen auf das Gesamtgewicht der Zubereitung.

Mittels der Mandelsäure, und den AT-wirkstoff - Aluminium-Salz - läßt sich überraschenderweise ein Hydrogel herstellen, dass die geforderten Eigenschaften, wie Transparenz und geringe Klebrigkeit aufweist. Darüber hinaus zieht die erfindungsgemäße Formulierung sehr schnell ohne Rückstände zu hinterlassen in die Haut ein. Tabelle 1 zeigt den Vergleich verschiedener transparenter Fomulierungen in einem Sensorik-Research-Panel, bestehend aus 8 geschulten Prüfern. Dazu wurden die Proben in definierter Menge auf die Haut aufgetragen und anhand einer Bewertungsskala bewertet (1 = nicht klebrig; 10 = stark klebrig).

**Tabelle 1**

| | **erfindungsgemäßes Beispiel** | **Vergleichsbeispiele** | | |
|---|---|---|---|---|
| | **Transparentes Hydrogel** | **Nanoemulsion** | **Wasser-in-Silikon-Emulsion** | **Wässrigalkoholische Formulierung** |
| **Einzugsvermögen in Sekunden** | 95 | 179 | 153 | 106 |
| **Klebrigkeit Skala von 1-10** | 3,4 | 5,2 | 6,5 | 5,3 |

Als besonders vorteilhaft hat sich eine Kombination aus Mandelsäure und Aluminium Clorohydrat gezeigt, wobei das Verhältnis Aluminum Chlorohydrat zu Mandelsäure 15:1 bis 1:1, bevorzugt 12:1 bis 2:1, insbesondere 10:1 bis 2,5:1, gewählt wird.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, UV-Filter, Antioxidantien, wasserlösliche Vitamine, Mineralstoffe, suspendierte Festkörperpartikel, Parfüme, Substanzen zum Verhindern des Schäumens, Farbstoffe, Pigmente, die eine färbende Wirkung haben, Verdickungsmittel, anfeuchtende und/oder feuchthaltende Substanzen oder andere übliche Bestandteile einer kosmetischen oder dermatologischen Formulierung wie Alkohole, Polyole, Polymere, Schaumstabilisatoren oder Silikonderivate.

Die Herstellung der erfindungsgemäß transparenten gelförmigen Zubereitung erfolgt vorteilhaft durch Lösen der Mandelsäure in Wasser. Anschließend erfolgt die Zugabe der wässrigen AT-Wirkstoffe, insbesondere Aluminum-Salz-Lösung, unter Rühren.

Zur Applikation der Zubereitung lassen sich herkömmlich Packmittel für Deodorantien und/oder Antitransipirantien verwenden, z. B. Stiftdispenser, Geldispenser, Tuben und Roller.
Angaben in Gewichtsprozent bezogen auf die Gesamtmasse der Zubereitung.

### Beispiele

| | 1 | 2 | 3 |
|---|---|---|---|
| Aluminum Chlorohydrat | 5 | 10 | 10 |
| Mandelsäure | 1,4 | 1,8 | 2 |
| Natriumcitrat | - | - | 1 |
| Wasser | 93,6 | 88,2 | 87 |
| Summe | 100 | 100 | 100 |

## Patentansprüche

1. Transparente kosmetische und/oder dermatologische Formulierung umfassend mindestens einen Antitranspirant-Wirkstoff, Mandelsäure und Wasser.

2. Formulierung nach Anspruch 1, **dadurch gekennzeichnet, dass** der Antitranspirant-Wirkstoff aus der Gruppe der Aluminium-Salze, bevorzugt Aluminium-Chlorohydrat oder Aluminium-Zirkonium-Salze, gewählt wird.

3. Formulierung nach einem der voranstehenden Ansprüche, **dadurch gekennzeichnet, dass** das Verhältnis Aluminium Chlorohydrat zu Mandelsäure im Bereich 15:1 bis 1:1, bevorzugt 12:1 bis 2:1, insbesondere 10 : 1, bis 2,5:1, gewählt wird.

4. Formulierung nach einem der voranstehenden Ansprüche, **dadurch gekennzeichnet, dass** der Antitranspirant-Wirkstoff in einer Menge von 1 bis 35 Gew.%, vorzugsweise von 1 bis 25 Gew. -%, besonders bevorzugt von 1 bis 20 Gew.% bezogen auf die Gesamtmasse der Formulierung, eingesetzt wird.

5. Formulierung nach einem der voranstehenden Ansprüche, **dadurch gekennzeichnet, dass** die Mandelsäure, in einer Menge von 0,1 bis 10 Gew.%, vorzugsweise von 0,1 bis 8 Gew.% bezogen auf die Gesamtmasse der Formulierung, eingesetzt wird.

6. Formulierung nach einem der voranstehenden Ansprüche, **dadurch gekennzeichnet, dass** die Formulierung eine definierte Fließgrenze aufweist.

7. Verwendung einer kosmetischen Formulierung nach mindestens einem der vorhergehenden Ansprüche zur Auftragung auf die menschliche Haut.

8. Verwendung einer Formulierung nach mindestens einem der Ansprüche 1 bis 6 zur Herstellung eines transparenten Deodorant- und/oder Antitranspirant-Hydrogels.

## Claims

1. Transparent cosmetic and/or dermatological formulation comprising at least one antiperspirant active ingredient, mandelic acid and water.

2. Formulation according to Claim 1, **characterized in that** the antiperspirant active ingredient is chosen from the group of aluminium salts, preferably aluminium chlorohydrate or aluminium zirconium salts.

3. Formulation according to one of the preceding claims, **characterized in that** the ratio of aluminium chlorohydrate to mandelic acid is chosen in the range 15:1 to 1:1, preferably 12:1 to 2:1, in particular 10:1 to 2.5:1.

4. Formulation according to one of the preceding claims, **characterized in that** the antiperspirant active ingredient is used in an amount of from 1 to 35% by weight, preferably from 1 to 25% by weight, particularly preferably from 1 to 20% by weight, based on the total mass of the formulation.

5. Formulation according to one of the preceding claims, **characterized in that** the mandelic acid is used in an amount of from 0.1 to 10% by weight, preferably from 0.1 to 8% by weight, based on the total mass of the formulation.

6. Formulation according to one of the preceding claims, **characterized in that** the formulation has a defined yield point.

7. Use of a cosmetic formulation according to at least one of the preceding claims for application to the human skin.

8. Use of a formulation according to at least one of Claims 1 to 6 for preparing a transparent deodorant and/or antiperspirant hydrogel.

## Revendications

1. Formulation cosmétique et/ou dermatologique transparente comprenant au moins un ingrédient actif anti-transpirant, de l'acide mandélique et de l'eau.

2. Formulation selon la revendication 1, **caractérisée en ce que** l'ingrédient actif anti-transpirant est choisi parmi le groupe constitué des sels d'aluminium, de préférence du chlorhydrate d'aluminium, ou des sels d'aluminium-zirconium.

3. Formulation selon l'une quelconque des revendications précédentes, **caractérisée en ce que** le rapport entre le chlorhydrate d'aluminium et l'acide mandélique est dans la plage de 15:1 à 1:1, de préférence de 12:1 à 2:1, en particulier de 10:1 à 2,5:1.

4. Formulation selon l'une quelconque des revendications précédentes, **caractérisée en ce que** l'ingrédient actif anti-transpirant est utilisé en une quantité de 1 à 35 % en poids, de préférence de 1 à 25 % en poids, particulièrement préférablement de 1 à 20 % en poids, par rapport à la masse totale de la formulation.

5. Formulation selon l'une quelconque des revendications précédentes, **caractérisée en ce que** l'acide mandélique est utilisé en une quantité de 0,1 à 10 % en poids, de préférence de 0,1 à 8 % en poids, par rapport à la masse totale de la formulation.

6. Formulation selon l'une quelconque des revendications précédentes, **caractérisée en ce que** la formulation présente une limite d'écoulement définie.

7. Utilisation d'une formulation cosmétique selon au moins l'une des revendications précédentes pour l'application sur la peau humaine.

8. Utilisation d'une formulation selon au moins l'une des revendications 1 à 6 pour la production d'un hydrogel déodorant et/ou anti-transpirant transparent.
